# EUROPEAN PATENT SPECIFICATION

(11) **EP 2 858 598 B1**
(45) Date of publication and mention of the grant of the patent: **10.04.2019**
(21) Application number: 13733470.2
(22) Date of filing: 28.05.2013
(51) Int. Cl.: A61F 2/18, B29C 45/00, C08K 3/00, A61F 11/00, B29L 31/00, B29B 9/06, B29C 33/72, B29K 23/00

(54) **FORMATION METHOD OF A MIDDLE EAR PROSTHESIS**
HERSTELLUNGSVERFAHREN EINER MITTELOHRPROTHESE
PROCÉDÉ DE FORMATION D'UNE PROTHÈSE D'OREILLE MOYENNE

(30) Priority: 06.06.2012 PL 39943912
(43) Date of publication of application: 15.04.2015
(73) Proprietor: Akademia Gorniczo-Hutnicza im. Stanislawa Staszica w Krakowie, 30-059 Krakow (PL)
(72) Inventor: ZIABKA, Magdalena, PL-32-080 Zabierzów (PL); CHLOPEK, Jan, PL-31-939 Kraków (PL); MERTAS, Anna, PL-44-103 Gliwice (PL); KRÓL, Wojciech, PL-42-600 Tarnowskie Góry (PL); MENASZEK, Elzbieta, PL-30-112 Kraków (PL); MORAWSKA, Agnieszka, PL-32-043 Ska a (PL)
(74) Representative: Plotczyk, Leokadia
(86) International application number: PCT/PL2013/000069
(87) International publication number: WO 2013/184010

(56) References cited:
- EP-A2- 0 456 135
- WO-A1-2012/036618
- US-A- 4 281 419
- US-A1- 2003 097 178
- US-A1- 2004 167 624
- US-A1- 2008 234 817
- US-A1- 2010 331 613
- US-B1- 6 228 324

## Description

The object of the invention is a method of producing middle ear prosthesis designed to recreate the sequence of ossicular chain.

Middle ear transmission is most frequently damaged as a result of chronic inflammation. Consequences of impaired middle-ear sound transmission include conductive hearing loss. This type of hearing loss may also occur as a result of mechanical damage incurred by specific elements of the ossicular chain or due to congenital deformities in these structures. Treatment of partly or completely damaged conducting apparatus of the middle ear involves surgery employing tympanoplastic techniques designed to repair the continuity of ossicular chain, i.e. malleus, incus, and stapes, by means of transplants or by adjusting and implanting alloplastic prostheses. The type of prosthesis depends on the condition of ossicles, the degree of their deformity and damage as well as the cause of their immobility, Prostheses known from both literature and patent descriptions, and used in medical practice, vary in terms of dimensions, shapes, and applied materials. Adequately designed material may trigger a different response to operation, and a modification in chemical composition allows for change in biological and mechanical parameters and prosthesis functions.

Total Ossicular Replacement Prosthesis (TORP) is used if it is necessary to recreate the entire sound conducting apparatus. TORP replaces all three ossicles. It is placed between the base of the stapes, which most frequently is undamaged, and the tympanic membrane or the transplant if it is necessary to perform myringoplasty (i.e. reconstruction of tympanic membrane). The prosthesis consists of a thin piston, equipped on one side with a support part, the so-called antenna connecting the prosthesis with the tympanic membrane and on the other with the so-called cup or bell which is attached to the head of the stapes, and if it is not there, on the arms of the stapes. The length of the piston depends on the size of the cavity as well as on the size of the patient's ossicles. The support part occurs in the form of a compact pad, or openwork antenna with diameter of approx. 2 mm. The functional structure of the antenna allows for precise placement of the implant during a surgery, which facilitates surgeon's work. The cup is in the shape of dome with an average height of 1 mm, the middle of which receives part of the healthy ossicle (e.g. head of the stapes). TORPs are known from e.g. American invention applications No. US20090164010 and US20110054607. Prostheses and their elements are made of titanium, gold, tantalum and their alloys, or from materials with shape memory, such as Nitinol, as well as ceramic materials or biocompatible polymers, in particular silicon or Polytetrafluoroethylene (PTFE) and composite materials containing carbon fibres. Previously known from American patent description No. US3909852, middle ear prosthesis contains a support part in the form of a pad made of Polytetrafluoroethylene supplemented with carbon fibres, and the piston is made of biocompatible polymers, such as e.g. Polytetrafluoroethylene or high-density polyethylene.

Partial Ossicular Replacement Prosthesis (PORP) is used if the conductive system retains at least on ossicle (malleus or stapes). These prostheses replace a part of the damaged conductive chain and are located between e.g. the malleus and the stapes. The polymer prosthesis known from Polish patent description No. PL156476 is used in the case of abnormal structure or dysfunction of the stapes. The fastening hook, called "crosier", made of metal (tantalum) makes it possible to hang the prosthesis on the long crus of the incus, and if there is no long crus or incus, on the first ossicle, and this way the transmission system is reconstructed. On the other hand, Australian patent application No. AU2011202466 proposes a solution where PORP consists of a piston, terminating on one side with an openwork antenna and on the other with a fastening clamp, the so-called boll, fixing the prosthesis to the preserved head of the stapes. Additionally, the mid part of the piston is enhanced with self-tightening tape, which simultaneously blocks and attaches the prosthesis to ossicles.

Introducing prosthesis into the organism during surgery should not require complicated implant procedures, and moreover, it should be possible to shape the prosthesis manually in order to adjust it specifically to the fragment of ossicular chain which is being replaced. Because of openwork (mesh-like) structure of the prosthesis, it is possible to visually control the process of introducing the implant precisely into the specific location within the middle ear. Due to the varied damage in the transmission chain, it is necessary to regulate and adjust the size of the prosthesis to the cavity during the surgery. It is possible to both adjust the prosthesis by bending the bell to the head of the stapes, and to regulate the length of the prosthesis which favourably impacts the process of adapting the implant to the parts of bones subject to replacement and facilitates the surgical procedure itself. Regulation can be performed in two sections of the prostheses. Metal prostheses can be adjusted within the segment defining the distance between the antenna and the bell. This type of regulated prosthesis is known, inter alia, from the German patent description No. DE102009016468. Its components, antenna and bell, are connected via an adjustable piston which makes it possible to extend the prosthesis along the axis. On the other hand in the case of polymer prostheses, because of the physical and chemical properties of such materials, the length of implants may be regulated by cutting the lower part of the prosthesis, i.e. the bell, and adjusting it to the part of the remaining bone. Another example of a middle ear prosthesis is disclosed in US2004/167624.

Advanced implants do not only promote the process of tissue regeneration but also improve and accelerate integration of implant with the bone, and frequently prevent recurrent diseases or inflammations due to adverse micro-organisms. These functions can be achieved by enriching the materials or by modifying and introducing bioactive, biostatic and biocidal additives. Previously known from American invention No. US20080234817 and American patent description No. US5578086, silicon and polyurethane prostheses are modified with biologically active hydroxyapatite ceramic or bioglass. Bioactive particles are dispersed in polymer matrix or occur on the surface of prosthesis, as coating. Other options include antibacterial coatings designed to reduce bacterial growth within the middle ear. These have been described, e.g. in a study by P. Gong, H. Li, X. He et al. entitled "Preparation and antibacterial activity of Fe3O4 and Ag nanoparticles" (Nanotechnology 18 (2007), 604-11). Such coatings contain e.g. nanoparticles of copper, zinc, titanium, magnesium, gold, as well as silver, the latter being most effective in preventing growth of bacteria, viruses and other microorganisms. American patent application No. US2011272276 focuses on coatings with antibacterial activity, releasing silver ions into human body in a controlled manner; these can be used in all kinds of implants introduced into the body. Materials used in prosthetics can also be enriched with pharmaceuticals. In accordance with American invention No. US20090088844 a possible solution is a prosthesis consisting of polymer piston and metal wire terminating with a hook. The piston contains a drug, which upon grafting into the body, is released into surrounding tissues in a controlled manner,

In accordance with the present invention, the method of producing middle ear prosthesis which involves formation of a profile made of polymer material by using injection technology, is characterized by the fact that thermoplastic polymer granulate is subject to a drying process at the temperature from 60 to 150°C for the duration of 2 to 8 hours, and then it is plasticized and injected in the temperature from 180 to 400°C, to a mould heated to the temperature from 60 to 150°C. The obtained profile is cooled down, and at the final stage is subjected to hermetic vacuum coating with film as well as low temperature plasma sterilization with the use of hydrogen peroxide vapour.

Preferably, high-density polyethylene, or polyamide or polysulfone are used as thermoplastic polymer.

Additionally, to be used as granulated thermoplastic polymer, the composite granules are obtained by introducing modifying bactericidal additive to the granulated thermoplastic polymer at wt. 0.1 - 3% which is followed by homogenization of the mixture, plastification, continuous extrusion, chilling and mechanical grinding.

The specific modifying bactericidal additive is silver powder with particle size in the range from 15 to 100 nm.

Middle ear prosthesis produced in accordance with the invention, where thermoplastic polymers are used as the material for reconstructing auditory ossicles, is low weight and its length may be adjusted to the length of the part of the conducting apparatus which is being replaced. The controlled length is obtained by cutting the prosthesis at the time of surgery. The applied openwork structure of the supporting part, i.e. the antenna, makes it possible to precisely and accurately implant the prosthesis during the operation, which significantly facilitates the surgeon's work, while the rounded profile of the antenna minimizes the risk of damaging the tympanic membrane. Additionally, as a result of introducing bactericidal additive in the form of silver nanoparticles into the polymer matrix, the middle ear prosthesis receives a new antimicrobial function against bacteria and fungi.

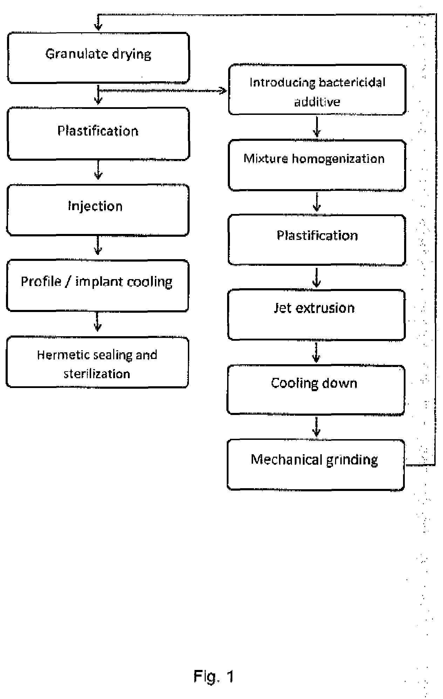

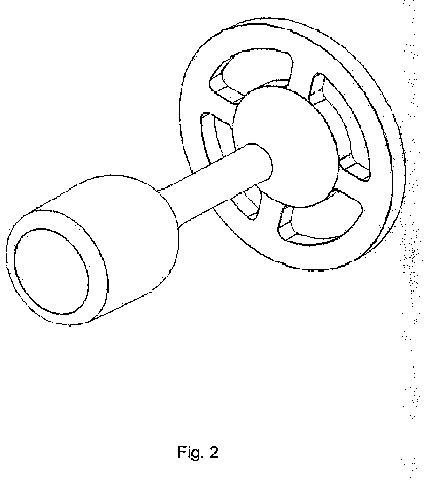

To enable its full understanding, the invention is explained with the description of an example of the technological process and a summary of activities in Fig. 1 of the drawing, while Fig. 2 presents the perspective view of the TOPR produced in accordance with the invention.

### Example 1, which does not form part of the present invention

Granulated high density polyethylene (HDPE) with molecular weight of 5000 g/mol and melting point in the range of 180 - 220°C is subject to drying process at the temperature of 100°C for 2 hours in a laboratory dryer. Then it is plasticized for 2 minutes in the chamber of injection moulding machine at the temperature of 200°C, after which it is injected at the temperature of 205°C, pressure of 80 kg/cm² and the flow rate of 60% to the mould heated to the temperature of 90°C. The obtained profile is cooled down to the temperature of approx. 60°C, retrieved from the mould and then all excess polymer is removed. At the final stage the profile is subjected to hermetic vacuum coating with film as well as low temperature plasma sterilization with the use of hydrogen peroxide vapour.

### Example 2

Granulated high density polyethylene (HDPE) with molecular weight of 5000 g/mol and melting point in the range of 180 - 220°C is subject to drying process at the temperature of 100°C for 2 hours in a laboratory dryer. Then silver nanopowder (Ag), with mean particle size of 26 nm, is added at wt. 2%. The mixture is homogenized by mechanical shaking for the duration of 20 minutes, and then plasticized and extruded continuously in the form of jet which is cooled down to ambient temperature and refined mechanically by grinder equipped with cutting blades. The obtained composite granulate is subject to a drying process at the temperature of 100°C for 2 hours in a laboratory dryer, then plasticized for 3 minutes in the chamber of injection moulding machine at the temperature of 200°C, after which it is injected at the temperature of 210°C, pressure of 80 kg/cm² and the flow rate of 60% to the mould heated to the temperature of 100°C, The obtained profile is cooled down to the temperature of approx. 60°C, retrieved from the mould and then all excess polymer is removed. At the final stage the profile is subjected to coating with film and to sterilization, like in Example 1. The simple structure of high density polyethylene and the well-distributed silver within its matrix significantly impact the quantity of ions released into the environment, thereby increasing antibacterial effectiveness of the composites. The effect has been confirmed with tests related to reference strains of Gram-positive bacteria (*Staphylococcus aureus*) and Gram-negative bacteria (*Escherichia coli*)*.* The findings are shown in the following chart.

| Material | *Staphylococcus aureus* ATCC 25923 (initial density 1.5 x 10⁵ CFU/ml) | | *Escherichia coli* ATCC 25922 (initial density 1.5 x 10⁵ CFU/ml) | |
|---|---|---|---|---|
| | Number of CFU/ml following 17-hour incubation with material | Bactericidal effectiveness ABE (%) | Number of CFU/ml following 17-hour incubation with material | Bactericidal effectiveness ABE (%) |
| HDPE | 5.6 x 10⁷ | 6.67 | 1.35 x 10⁸ | 10.0 |
| HDPE/wt 2% Ag | 1.0 x 10⁴ | 99.9 | 1.4 x 10³ | 99.9 |
| BLANK | 6.0 x 10⁷ | --- | 1.5 x 10⁸ | --- |

| | | | | |
|---|---|---|---|---|
| *CFU - Colony Forming Units (refers to the applied bacterial suspension in tryptone water, with the cellular density of bacteria forming colonies). | | | | |

## Claims

1. A method of producing middle car prosthesis involving formation of a profile made of polymer material by using injection technology, where a thermoplastic polymer granulate after process of drying at a temperature from 60 to 150°C for a duration of 2 to 8 hours is plasticized and injected at a temperature from 180 to 400°C to a mould preheated to a temperature from 60 to 150°C,
while at the final stage the profile itself is subjected to hermetic vacuum coating with a film as well as low temperature plasma sterilization with a hydrogen peroxide vapour, wherein
the polymer granulate before the processes of drying and uploading to the injection mould machine is subjected to an additional treatment, where the previously dried thermoplastic polymer granulate is modified by addition into whole volume the bactericidal additive in amount of 0.1 up to 3 wt. % of the total weight, and further
the mixture is subjected to homogenisation, plastification, continuous extrusion, chilling, and finally, mechanical grinding.

2. The method of claim 1 wherein high-density polyethylene, or polyamide or polysulfone are used as the thermoplastic polymer.

3. The method of claim 2 wherein the modifying bactericidal additive is silver powder with particle size in a range from 15 to 100 nm.

## Patentansprüche

1. Ein Verfahren zur Herstellung einer Mittelohrprothese, das die Bildung eines Profils aus Polymermaterial unter Verwendung einer Injektionstechnologie umfasst, wobei ein thermoplastisches Polymergranulat nach einem Trocknungsprozess bei einer Temperatur von 60 bis 150°C für eine Dauer von 2 bis 8 Stunden plastifiziert wird und bei einer Temperatur von 180 bis 400°C in eine auf eine Temperatur von 60 bis 150°C vorgewärmte Form eingespritzt wird, während das Profil selbst in der Endphase einer hermetischen Vakuumbeschichtung mit einem Film sowie einer Niedertemperatur-Plasmasterilisation mit einem Wasserstoffperoxiddampf unterzogen wird, wobei das Polymergranulat vor dem Trocknungsprozess und Hochladen in die Spritzgießmaschine einer zusätzlichen Behandlung unterzogen wird, wobei das zuvor getrocknete thermoplastische Polymergranulat durch Zugabe des bakteriziden Zusatzstoffes in einer Menge von 0,1 bis zum 3 Gew. % des Gesamtgewichts modifiziert wird, und ferner das Gemisch der Homogenisierung, Plastifizierung, kontinuierliches Extrudieren, Abkühlen und schließlich mechanischen Schleifen unterzogen wird.

2. Verfahren nach Anspruch 1, wobei als thermoplastisches Polymer Polyethylen hoher Dichte oder Polyamid oder Polysulfon verwendet wird.

3. Verfahren nach Anspruch 2, wobei der modifizierende bakterizide Zusatzstoff Silberpulver mit einer Teilchengröße im Bereich von 15 bis 100 nm ist.

## Revendications

1. Une méthode de production d'une prothèse d'oreille moyenne impliquant la formation d'un profilé en matériau polymère en utilisant la technologie d'injection, où un granulé de polymère thermoplastique, après le processus de séchage à une température de 60 à 150°C pendant une durée de 2 à 8 heures, est plastifié et injecté à une température de 180 à 400°C dans un moule préchauffé à une température de 60 à 150°C, tandis qu'au stade final, le profilé lui-même est soumis à un revêtement hermétique sous vide avec un film ainsi qu'à une stérilisation au plasma à basse température avec une vapeur de peroxyde d'hydrogène, dans laquelle le granulat de polymère avant le processus de séchage et de chargement dans la machine de moulage par injection est soumis à un traitement supplémentaire, où le granulat de polymère thermoplastique préalablement séché est modifié par addition en volume entier de l'additif bactéricide en une quantité de 0,1 à 3% en poids du poids total, et en outre, le mélange est soumis à une homogénéisation, une plastification, une extrusion continue, un refroidissement et enfin un broyage mécanique.

2. La méthode selon la revendication 1, dans laquelle le polyéthylène haute densité ou le polyamide ou le polysulfone sont utilisés comme le polymère thermoplastique.

3. La méthode selon la revendication 2, dans laquelle l'additif bactéricide modificateur est une poudre d'argent dont la taille des particules varie de 15 à 100 nm.
